# EUROPEAN PATENT SPECIFICATION

(11) **EP 1 234 887 B1**
(45) Date of publication and mention of the grant of the patent: **28.09.2005**
(21) Application number: 01104787.5
(22) Date of filing: 27.02.2001
(51) Int. Cl.: C12Q 1/68

(54) **Process for binding nucleic acids to a carrier**
Verfahren zur Bindung von Nukleinsäuren an einen Träger
Procédé pour attacher acides nucléiques sur un support

(43) Date of publication of application: 28.08.2002
(73) Proprietor: Deutsches Krebsforschungszentrum Stiftung des Öffentlichen Rechts, 69120 Heidelberg (DE)
(72) Inventor: Diehl, Frank, 69115 Heidelberg (DE); Grahlmann, Susanne, 69115 Heidelberg (DE)
(74) Representative: Störle, Christian

(56) References cited:
- EISEN MICHAEL B ET AL: "DNA arrays for analysis of gene expression." METHODS IN ENZYMOLOGY, vol. 303, 1999, pages 179-205, XP000995864 1999 Academic Press, Inc.; Academic Press Ltd. 1250 Sixth Ave., San Diego, California 92101, USA; 14 Belgrave Square, 24-28 Oval Road, London NW1 70X, England, UK ISBN: 0-12-182204-4
- CHEUNG, V.G. ET AL.: "Making and reading microarrays" NATURE GENETICS, vol. 21, no. suppl, January 1999 (1999-01), pages 15-19, XP000996654
- LIPSHUTZ, R.J. ET AL.: "High density synthetic oligonucleotide arrays" NATURE GENETICS, vol. 21, no. suppl, January 1999 (1999-01), pages 20-24, XP000996655
- REES WILLIAM A ET AL: "Betaine can eliminate the base pair composition dependence of DNA melting." BIOCHEMISTRY, vol. 32, no. 1, 1993, pages 137-144, XP000602183 ISSN: 0006-2960
- HENKE WOLFGANG ET AL: "Betaine improves the PCR amplification of GC-rich DNA sequences." NUCLEIC ACIDS RESEARCH, vol. 25, no. 19, 1997, pages 3957-3958, XP002171292 ISSN: 0305-1048
- DIEHL, F. ET AL.: "Manufacturing DNA microarrays of high spot homogeneity and reduced background signal" NUCLEIC ACIDS RESEARCH, vol. 29, no. 7, 1 April 2001 (2001-04-01), page e38 XP002171293

## Description

The invention relates to a process for binding nucleic acids to a carrier.

DNA chip technology is a research area which is still under development and wherein DNA chips having up to several thousand spots are used. To this end, nucleic acids are fixed on a carrier in an orderly grid pattern. The DNA or RNA to be examined is labelled, e.g. using a fluorescent dye, and applied to the chip. In the case of hybridization to sensor molecules bound to carriers having complementary sequences, a signal is detected at a corresponding position within said grid through a CCD camera or by a laser scanner.

For the fabrication of microarrays, the nucleic acids to be bound to carriers are stored in microtiter plates. For the spotting process, the nucleic acids are only solved in a very small volume, for instance 5 to 10 µl. The spotting solution, in which the nucleic acids are dissolved, is spotted on the carrier, generally glass slides. When nucleic acids dissolved in a solvent are deposited on a glass surface, the solvent evaporates quickly leading to an unequal attachment of nucleic acids to the surface of the glass and a low binding efficiency to the glass.

As a spotting solution, saline sodium citrate (SSC) buffer is widely used. However, binding efficiency and spot uniformity are very poor when using this buffer. The problems are reduced by supplementing SSC with 50% dimethyl sulfoxide. This substance has the disadvantage, however, of being both toxic and a solvent of many materials, apart from its only limited effect on spot appearance.

Thus, the technical problem underlying the present invention is to provide a process for binding nucleic acids to a carrier not having the disadvantages of the processes of the prior art.

According to the present invention, this technical problem is solved by a process for binding nucleic acids to a carrier wherein the nucleic acids are dissolved in a solvent containing at least one compound selected from the group consisting of betaines having the atom grouping R₃N^{⊕}-CH₂COO^{ψ} (IUPAC Rule C-816.1), wherein the group R can be independently selected from C₁-C₅ alkyl, in particular methyl, ethyl and propyl, the obtained solution being applied to a carrier and the nucleic acids being bound to the carrier.

The term "nucleic acids" as used herein refers to either DNA or RNA. It includes plasmides, infectious polymers of DNA and/or RNA, non-functional DNA or RNA, chromosomal DNA or RNA, and DNA or RNA synthesized *in vitro,* such as by the polymerase chain reaction or oligonucleotide sythesis. For a person skilled in the art, it is well-known to produce DNA or RNA *in vitro,* in particular by the polymer chain reaction.

According to the process of the present invention, the solvent in which the nucleic acids are dissolved contains at least one compound selected from the group consisting of betaines. The betaines can be present in the solvent before the nucleic acids are dissolved. On the other hand, the betaines can be added to the solvent after the nucleic acids are dissolved therein.

In a preferred embodiment of the present invention, the compound selected from the group consisting of betaines is trimethylammonium acetate (betaine) since a particularly good binding of nucleic acids to the carrier is achieved with it.

Preferably, the compound selected from the group consisting of betaines is present in said solvent at a concentration of 8 mM to 6.5 M, in particular 1.5 M, since in this concentration range good effects of the betaines on the binding of nucleic acids to the carrier were observed.

As mentioned above, the nucleic acids are dissolved in a solvent. The term "solvent" as used herein refers to any liquid capable of dissolving nucleic acids. Generally, the main ingredient of such liquids is water, which can be buffered to a certain pH value.

If the process according to the invention is used for manufacturing microarrays, so-called spotting solutions can be used as solvent. Examples of the spotting solutions are saline sodium citrate (SSC) buffer, in particular containing 45 mM Na-citrate, 450 mM NaCI, pH 7.0 (3xSSC).

In the process according to the present invention, the nucleic acids-containing solvent is applied to a carrier, and the nucleic acids are bound to the carrier.

Preferably, the carrier is made of glass, in particular slides, due to its favourable optical characteristics. Furthermore it is solid, planar and not fluorescent.

The binding of the nucleic acids to the carrier can be achieved either by covalent or electrostatical (ionical) bounds.

For covalent binding, for example compounds providing aldehyde groups are coated on the surface of the carrier. Nucleic acids contain primary amino groups which can react with the aldehyde group to form a Schiff base, i.e. a covalent bond.

For the electrostatical binding, use is made of the fact that nucleic acids are generally negatively charged. By providing positive charges on the surface of the carrier, binding between the negatively charged nucleic acids and the positively charged surface of the carrier can be achieved by an interaction of the charges. For this purpose, glass surfaces coated with compounds providing positive charges, e.g. coated with poly-L-lysine and/or aminosilane, are used. Such activated slides are well-known in the art.

If necessary, cross-linking of the nucleic acids with the carrier can be achieved by UV irradiation, for example with a total energy of 60 mJ.

It has to be understood that the process according to the present invention can be carried out in a wide variety of methods in which nucleic acids are bound to carriers. In particular, the process of the present invention is generally useful for the fabrication of microarrays which is known from the prior art. The following illustrates the fabrication of microarrays using the process of the present invention, but it has to be understood that the present invention is not limited to the fabrication of microarrays.

Poly-L-lysine-coated glass slides, e.g. of 75 mm x 25 mm, can be prepared as described by Schena, M. et al., (1995) Quantitative Monitoring of Gene Expression Patterns with a Complementary DNA Microarray, Science, 467-470. Slides having an aminosilane surface are commercially available (for example CMT-GAPS™ from Corning, USA). The DNA spotting solution can be adjusted to 45 mM Na-citrate, pH 7.0, 450 mM NaCI (3xSSC) containing, e. g. 1.5 M betaine. Varying the parameters, it was found that a concentration of 1.5 M betaine had the overall best effect on the quality of DNA microarrays. DNA spotting can usually be done, for instance, with an SDDC-2 DNA Micro Arrayer from Engineering Services Inc. (Toronto, Canada). A single SMP3 pin (Telechem International Sunnyvale, USA) can be used to avoid differences between the pins. The DNA samples can be printed in quadruplicate at a 200 µm centre-to-centre spacing. Slides can be left at room temperature for several hours, e.g. 10-20 hours, and then heat-treated on a metal block at about 80°C for about 5 seconds. The DNA can be cross-linked to the support by UV-irradiation with a total energy of about 60 mJ using a commercially available UV-crosslinker, for instance a Hoefer UV-crosslinker (Amersham Pharmacia Biotech, Freiburg, Germany).

The process of the present invention has a plurality of advantages. Analysis of bound nucleic acids, in particular on DNA-microarrays, depends considerably on spot quality and low background signal of the glass support. By using at least one compound selected from the group consisting of betaines as an additive to a solvent, in particular spotting solutions, for dissolving nucleic acids both the binding efficiency of spotted PCR products and the homogenity of the DNA spots are improved significantly, in particular by using aminated surfaces, such as glass slides coated with the widely used poly-L-lysine and/or aminosilane. The more nucleic acids are bound during this process, the better the subsequent analysis will be, since the efficiency of binding nucleic acids to glass slides limits the sensitivity and the dynamic range of such measurements. Also, nucleic acid spots of high homogenity are beneficial, since they simplify image analysis and enhance considerably the accuracy of signal detection. In addition, unspecific background signal is markedly diminished. Furthermore, the betaines reduce evaporation from the microtiter dish wells during the array procedure, which hold the nucleic acids.

An important part of microarray manufacturing is the processing of the glass surface after spotting the nucleic acids, during which the remaining, unreacted amino residues of the poly-L-lysine polymer and/or aminosilane are deactivated. This prevents subsequent binding of nucleic acids, which increases the background signal upon hybridisation of a labelled target. The blockage can be achieved by various methods of the prior art, for example by reacting the arrays with succinic anhydride in aqueous borate-buffered 1-methyl-2-pyrrolidinone, converting the amines into carboxylic moieties. During this process, however, the spotted nucleic acids come into contact with the aqueous blocking solution, are partly re-dissolved and spread across the entire slide.

To prevent this, a robust processing method has been developed wherein the slides are treated with a solution of succinic anhydride as a blocking agent and an acylating catalyst in an unpolar non-aqueous solvent.

Preferably, said acylating catalyst is N-methylimidazol, since it is highly soluble, has low toxicity and functions as a buffer to maintain a pH of about 8. It is further preferred that the unpolar non-aqueous solvent is 1,2-dichloroethane, which has the advantage that it is a good solvent for both the succinic anhydride and the N-methylimidazol.

A preferred composition for the blocking solution contains 0.2 g to 20 g, in particular about 1 g, of succinic anhydride and 1 ml to 10 ml, in particular about 2.5 ml, of N-methylimidazol dissolved in about 200 ml of 1,2-dichloroethane. In order to achieve good results, the molar ratio of N-methylimidazol to succinic anhydride can be 2:1 to 4:1, in particular about 3:1.

A detailed example of the blocking process is given hereinafter: about 1 g of succinic anhydride is freshly dissolved in 200 ml of anyhdrous 1,2-dichloroethane. To this solution, about 2.5 ml of N-methylimidazol can be added and immediately poured into a slide chamber. Incubation of the slide in this solution is carried out for about 1 hour, placed on an orbital shaker for slide agitation. Subsequently, the slides can be washed briefly in about 200 ml of fresh 1,2-dichloroethane and incubated in boiling water for 2 minutes for nucleic acid denaturation. After a brief rinse in 95% ethanol, they can be left to dry at room temperature.

Surprisingly, it has been found that blocking of the chip surface with succinic anhydride in an unpolar non-aqueous solvent, in particular 1,2-dichloroethane, in the presence of an acylating catalyst, in particular N-methylimidazol, prevents overall background signal that occurs with the frequently applied aqueous solvent 1-methyl-2-pyrrolidon in borate buffer.

As is evident from the foregoing, betaines are useful in processes for binding nucleic acids to carriers, wherein the betaines are present as additives in the solvent, in which the nucleic acids are dissolved before applying them to the carrier and binding them to it. Accordingly, the use of betaines as additives for solvents in which nucleic acids are dissolved in order to bind them to a carrier is a further subject of the present invention, wherein the terms "betaines", "solvents", "nucleic acids" and "carrier" are defined as above.

Short description of the drawings:
- Figure 1: shows signal intensities produced upon hybridisation of Cy5-labelled DNA to increasing amounts of spotted PCR-product;
- Figure 2: shows the effect of probe concentration and spotting solution on hybridisation efficiency;
- Figure 3: shows the comparison of blocking reactions; and
- Figure 4: shows the effect of betaine on evaporation.

The following examples serve to illustrate the invention in more detail.

### Example 1: Manufacturing of DNA-microarrays

### a) Probe and target synthesis

Non-homologous DNA-inserts of about 500 bp length were picked at random from a clone library generated by cDNA representational difference analysis (Hubank, M. and Schatz, D.G. (1994) Identifying differences in mRNA expression by representational difference analysis of cDNA, Nucleic Acids Res., 22, 5640-5648). They were PCR-amplified in 100 µl reactions with the universal primer d(AGGCAACTGTGCTATCCGAGGGAA), purified by isopropanol precipitation and resuspended in water. The DNA-concentration was determined by measuring the fluorescence signal obtained in the presence of the dye Hoechst-33258. Purity of the fragments was checked by agarose gel electrophoresis. For the generation of complementary hybridisation targets, a Cy5-labelled oligonucleotide primer of identical sequence was used for amplification.

### b) Fabrication of microarrays

Poly-L-lysine-coated glass slides of 75 mm x 25 mm were prepared as described by Schena, M. et al., Quantitative Monitoring of Gene Expression Patterns with a Complementary DNA Microarray, Science, 270, 467-470. Slides having an aminosilane surface (CMT-GAPS™) were purchased from Coming (Corning, USA). The DNA spotting solution was adjusted to either 45 mM Na-citrate, pH 7.0, 450 mM NaCI (3xSSC) or the same composition was used supplemented with 1.5 M betaine (obtained from Sigma, Germany). DNA spotting was done with an SDDC-2 DNA Micro-Arrayer from Engineering Services Inc. (Toronto, Canada). A single SMP3 pin (TeleChem International Inc., Sunnyvale, USA) was used to avoid differences between pins. The DNA samples were printed in quadruplicate at a 200 µm centre-to-centre spacing. Slides were left at room temperature overnight and then heat-treated on a metal block at 80°C for 5 sec. The DNA was cross-linked to the support by UV-irradiation with a total energy of 60 mJ in a Hoefer UV-crosslinker (Amersham Pharmacia Biotech, Freiburg, Germany). For the blocking process, 1 g of succinic anhydride (Fluka, Deisendorf, Germany) was freshly dissolved in 200 ml of anhydrous 1,2-dichloroethane (Fluka). To this solution, 2.5 ml of N-methylimidazol (Fluka) was added and immediately poured into the slide chamber. Incubation in this solution was for 1 hour, placed on an orbital shaker for slight agitation. Subsequently, the slides were briefly washed in 200 ml of fresh 1,2-dichloroethane and incubated in boiling water for 2 min for DNA denaturation. After a brief rinse in 95% ethanol, they were left to dry at room temperature.

### c) Hybridisation of labelled samples

For each hybridisation, 0.2 µg of Cy5-labelled and 1.8 µg of unlabelled PCR-product were mixed and precipitated with ethanol. The pellet was taken up in 15 µl hybridisation buffer of 50% formamide, 3xSSC, 1% SDS, 5xDenhardt's reagent and 5% dextran sulfate. The sample was denatured at 80°C for 10 min, applied to a microarray and spread evenly by a coverslip of 22 mm x 22 mm. Hybridisation was done for 16 h at 42°C in a humidified hybridisation chamber (TeleChem International Inc.). The slides where washed in 2xSSC, 0.1% SDS for 2 min, then in 1xSSC for 2 min, rinsed briefly in 0.2xSSC and dried by centrifugation at 500 rpm for 5 min. Detection of the fluorescence signals was performed on a ScanArray5000 unit and analysed with the QuantArray1.0 software package (GSI Lumonics, Billerica, USA).

### d) Effectiveness of DNA binding

One critical factor in microarray analysis is the amount of probe material attached to the support that is available for hybridisation. This factor can quickly limit the signal intensities detectable on glass arrays, and thus it directly influences the sensitivity and dynamic range of measurements. In order to determine how the buffer condition of the spotting solution affects the binding efficiency of the spotted DNA, PCR-products of about 500 bp in length were produced from individual clone inserts, which had been randomly picked from a subtractive human clone library (cf. above). The DNA was diluted to concentrations of 500 ng/µl, 250 ng/µl, 100 ng/µl, 50 ng/µl and 25 ng/µl and applied to glass slides in four replica-spots each (cf. above). Also, spotting solution without DNA was deposited. Parallel to 3xSSC and the same buffer supplemented with 1.5 M betaine, the commercial Arraylt™ micro-spotting solution of TeleChem International Inc. was tested.

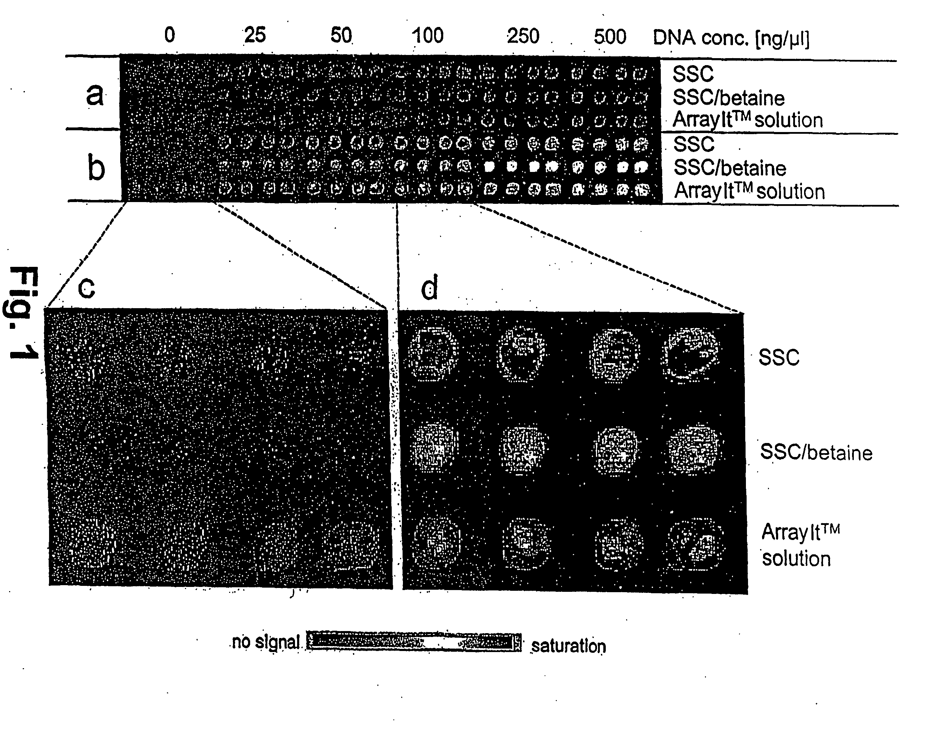

Labelled PCR-products were hybridised. Figure 1 shows a typical image of fluorescence signal intensities obtained from such experiments. Spots obtained with each DNA concentration and buffer system were present in quadruplicate. Signals in (a) represent the background of non-specific binding to a non-complementary sequence. In (b), the signals obtained on a fully complementary probe are shown. Panels (c) and (d) represent enlargements that display in detail the background signal collected in the absence of DNA (c) and the homogeneity of signal for spots produced with 100 ng/µl DNA (d).

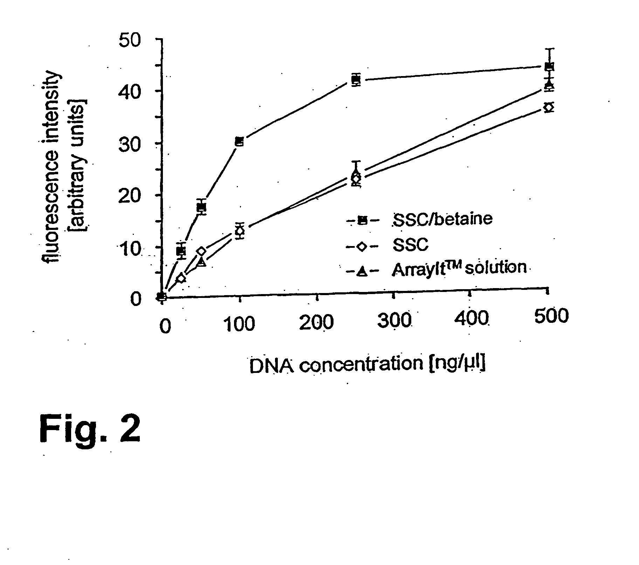

Irrespective of the buffer, hybridisation was specific to the complementary probe molecule. Also, in all cases the signal intensities increased with increasing concentration of the spotted DNA-probe solution. However, quantification reveals that at a DNA-concentration in the spotting solution of up to 100 ng/µl the signal intensities were higher by a factor of about 2.5, where betaine had been present in the spotting buffer (Fig. 2). Fig. 2 shows the effect of probe concentration and spotting solution on hybridisation efficiency. The mean signal intensities produced in the experiments shown in Fig. 1 are plotted versus the DNA concentration of the spotted DNA. The error bars indicate the standard deviation. Correspondingly, the binding capacity of the glass surface is nearly saturated at a DNA concentration of 250 ng/µl with betaine, while without betaine this level is reached only at a concentration higher than 500 ng/µl.

### e) Spot homogeneity

Spot homogeneity is dependent on the variation of the DNA-concentration across a spot. There are distinct, frequently occurring patterns that can be observed upon hybridisation, such as a higher DNA concentration at the edges ("doughnut" effect) or the aggregation of the DNA at few points within a spot. The former effect was seen on slides printed with DNA in pure SSC buffer, while the latter occurred when the Arraylt™ micro-spotting solution was used (Fig. 1). Supplementing SSC with 1.5 M betaine yielded much more homogenous spots. This effect was evaluated by calculating the variation coefficient of signal intensity across all pixels that represent a spot. At a DNA concentration of 100 ng/µl during spotting (Fig. 1 d), for example, the variation coefficient was found to be 7% with the commercial buffer, 14% if SSC was used and only 5% for SSC supplemented with betaine.

### f) Spot-specific background signal

The choice of spotting solution also has a noticeable effect on the background signal produced at the spots in absence of a complementary target DNA. In Fig. 1 c, typical results are presented, if a buffer without any DNA has been spotted. Particular care was taken to avoid any carry-over of DNA from other samples by extensive washing steps and spotting the buffer probe first before proceeding to samples containing DNA. The signal-to-noise ratio of each feature was calculated by dividing the mean signal intensity of the four spot areas by the mean of the background signal in between spots. A ratio of 0.7 (±0.2) was found for 3xSSC supplemented with 1.5 M betaine, while much higher ratios of 5.1 (±0.8) and 10.5 (±1.5) were determined for SSC without betaine and the TeleChem Arraylt™ micro-spotting solution, respectively.

### Example 2: Suppression of overall background during manufacturing of DNA microarrays

The microarrays were manufactured as described in Example 1 and the suppression of overall background caused by the postprocessing was evaluated, wherein the blocking process of the prior art is compared with the effect of the blocking process according to the present invention.

The method of slide postprocessing with succinic anhydride was introduced by Schena et al. (Quantitative Monitoring of Gene Expression Pattern with Complementary DNA Microarray, Science, 270, 467-470) and is widely used for the blocking of aminated surfaces by acylating the unreacted primary amines. In this process, succinic anhydride is first dissolved in 1-methyl-2-pyrrolidone (NMP) before sodium borate buffer (pH 8) is added; the final concentrations are 164 mM succinic anhydride, 96% (v/v) NMP and 4% (v/v) aqueous sodium borate buffer.

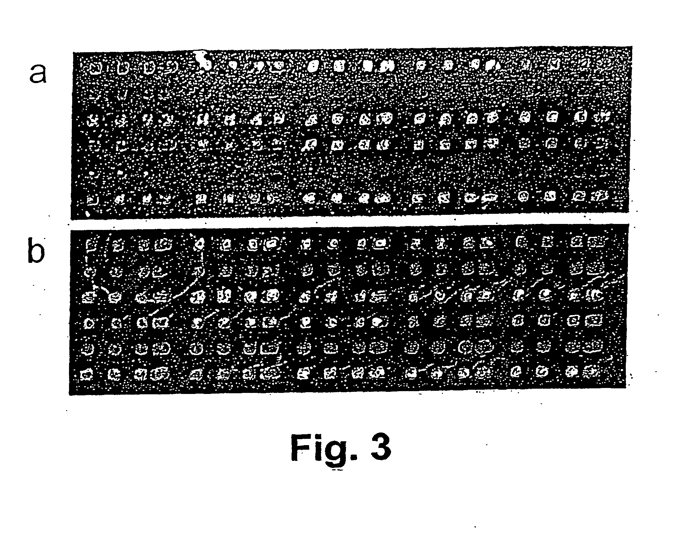

It is assumed that incubation in this solution re-dissolves part of the DNA deposited on the glass surface, which then could spread across the slide causing additional background. In an effort to avoid this effect, the unpolar, non-aqueous solvent 1,2-dichloroethane (DCE) was substituted for NMP, see example 1 for details. The concentration of succinic anhydride was decreased to 50 mM. Also, no aqueous buffer was added to the solution. Instead, the acylating catalyst N-methylimidazol was added for acceleration of the process. Slides produced and processed parallel but acylated by either the NMP-method or DCE-method of the present invention were compared. With the latter blocking reaction of the present invention, an overall significantly reduced background is achieved (Fig. 3). Fig. 3 shows microarray slides produced simultaneously before being subjected to the blocking procedures. In (a), acylation was performed by 164 mM succinic anhydride in borate buffered NMP, while in (b) 50 mM succinic anhydride and 150 mM N-methylimidazol in DCE were used. The slides were hybridised in parallel with a Cy5-labelled, complementary PCR-product, briefly washed and scanned under identical conditions. The slight DNA "tails" seen in (b) are caused by target DNA left after the brief washing. Such features occur on both types of slides, as was determined by radioactive hybridisations, but are submerged in the background signal of slide (a). Ever since using the DCE-based process (present invention) as a blocking procedure, no background problems attributed to the blocking have ever been encountered, whereas before, when using the NMP-method of the prior art, all commonly known problems were experienced, such as inverted signal phenomena or a higher background around DNA-spots.

### Example 3: Effect of betaine on evaporation

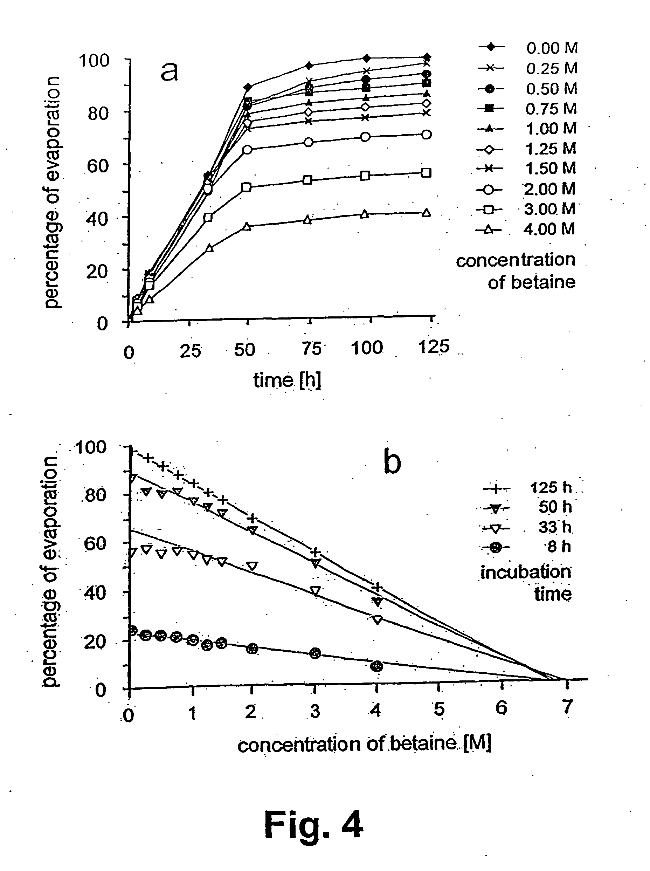

In order to determine the effect of betaine on evaporation, 1 ml of spotting solution supplemented with different concentrations of betaine was filled in a 1.5 ml Eppendorf cup, which was incubated with an open lid at 30°C. The results are shown in Fig. 4. In panel (a), the percentage of evaporation is presented. It is pointed out that by the increase in betaine concentration at the liquid surface the evaporation eventually stops. From this data, it can be extrapolated (b) that betaine at a concentration of 6.8 M prevents further evaporation.

## Claims

1. A process for binding nucleic acids to a carrier, wherein the nucleic acids are dissolved in a solvent containing at least one compound selected from the group consisting of betaines having the atom grouping R₃N⁺-CH₂-COO⁻, wherein the group R is independently selected from C₁-C₅ alkyl, the obtained solution being applied to a carrier and the nucleic acids being bound to the carrier.

2. The process according to claim 1, wherein the compound selected from the group consisting of betaines is trimethylammonium acetate.

3. The process according to claim 1 or 2, wherein the compound selected from the group consisting of betaines is present in said solvent at a concentration of 8 mM to 6.5 M.

4. The process according to one of the preceding claims, wherein the solvent contains about 1.5 M of sodium chloride and about 150 mM of sodium citrate, and wherein the pH value is about 7.

5. The process according to one of the preceding claims, wherein said carrier is made of glass.

6. The process according to claim 5, wherein said glass is coated with poly-L-lysine and/or an aminosilane.

7. The process according to claim 6, wherein said glass, after binding of the nucleic acids thereto, is treated in order to deactivate the poly-L-lysine and/or the aminosilane.

8. The process according to claim 7, wherein said glass is treated with a solution of succinic anhydride as blocking agent and an acylating catalyst in an unpolar non-aqueous solvent.

9. The process according to claim 8, wherein said acylating catalyst is N-methylimidazol.

10. The process according to one of claims 8 or 9, wherein the unpolar non-aqueous solvent is 1,2-dichloroethane.

11. The process according to one of claims 8 to 10, wherein 0.2 g to 20 g of succinic anhydride and 1 ml to 10 ml of N-methylimidazol are dissolved in about 200 ml of 1,2-dichloroethane.

12. The use of betaines as additives for solvents in which nucleic acids are dissolved in order to bind them to a carrier, wherein the betaines have the atom grouping R₃N⁺-CH₂-COO⁻, wherein the group R is independently selected from C₁-C₅ alkyl.

## Patentansprüche

1. Verfahren zum Binden von Nukleinsäuren an einen Träger, wobei die Nukleinsäuren in einem Lösungsmittel gelöst sind, das mindestens eine Verbindung enthält, die aus der Gruppe ausgewählt ist, welche aus Betainen mit der Atomgruppe R₃N⁺-CH₂-COO besteht, worin die Gruppe R unabhängig unter C₁-C₅-Alkyl ausgewählt ist, wobei die erhaltene Lösung auf einen Träger aufgebracht wird und die Nukleinsäuren an den Träger gebunden werden.

2. Verfahren nach Anspruch 1, bei dem die Verbindung, die aus der Gruppe ausgewählt ist, die aus Betainen besteht, Trimethylammoniumacetat ist.

3. Verfahren nach Anspruch 1 oder 2, bei dem die Verbindung, die aus der Gruppe ausgewählt ist, welche aus Betainen besteht, in einer Konzentration von 8 mM bis 6,5 M in dem Lösungsmittel vorhanden ist.

4. Verfahren nach einem der vorhergehenden Ansprüche, bei dem das Lösungsmittel etwa 1,5 M Natriumchlorid und etwa 150 mM Natriumcitrat enthält und der pH-Wert etwa 7 beträgt.

5. Verfahren nach einem der vorhergehenden Ansprüche, bei dem der Träger aus Glas besteht.

6. Verfahren nach Anspruch 5, bei dem das Glas mit Poly-L-lysin und/oder einem Aminosilan beschichtet ist.

7. Verfahren nach Anspruch 6, bei dem das Glas nach dem Binden der Nukleinsäuren daran behandelt wird, um das Poly-L-lysin und/oder Aminosilan zu deaktivieren.

8. Verfahren nach Anspruch 7, bei dem das Glas mit einer Lösung von Bernsteinsäureanhydrid als Blockiermittel und einem Acylierungskatalysator in einem unpolaren, nicht-wäßrigen Lösungsmittel bebandelt wird.

9. Verfahren nach Anspruch 8, bei dem der Acylierungskatalysator N-Methylimidazol ist.

10. Verfahren nach einem der Ansprüche 8 oder 9, bei dem das unpolare, nicht-wäßrige Lösungsmittel 1,2-Dichlorethan ist.

11. Verfahren nach einem der Ansprüche 8 bis 10, bei dem 0,2 g bis 20 g Bernsteinsäureanhydrid und 1 ml bis 10 ml N-Methylimidazol in etwa 200 ml 1,2-Dichlorethan gelöst werden.

12. Verwendung von Betainen als Additive für Lösungsmittel, in denen Nukleinsäuren gelöst sind, um diese an einen Träger zu binden, wobei die Betaine die Atomgruppe R₃N⁺-CH₂-COO⁻ aufweisen, worin die Gruppe R unabhängig unter C₁-C₅-Alkyl ausgewählt ist.

## Revendications

1. Procédé pour lier des acides nucléiques à un support, dans lequel les acides nucléique sont dissous dans un solvant contenant au moins un composé choisi parmi le groupe constitué de bétaïnes ayant le groupement d'atome R₃N⁺-CH₂-COO⁻, dans lequel le groupe R est indépendamment choisi parmi un alkyle en C₁ à C₅, la solution obtenue étant appliquée à un support et les acides nucléiques étant liés au support.

2. Procédé selon la revendication 1, dans lequel le composé choisi parmi le groupe constitué de bétaïnes est l'acétate de triméthylammonium.

3. Procédé selon la revendication 1 ou 2, dans lequel le composé choisi parmi le groupe constitué de bétaïnes est présent dans ledit solvant à une concentration de 8 mM à 6,5 M.

4. Procédé selon l'une quelconque des revendications précédentes, dans lequel le solvant contient environ 1,5 M de chlorure de sodium et environ 150 mM de citrate de sodium et dans lequel la valeur du pH est d'environ 7.

5. Procédé selon l'une quelconque des revendications précédentes, dans lequel ledit support est fait de verre.

6. Procédé selon la revendication 5, dans lequel ledit verre est revêtu de poly-L-lysine et/ou d'un aminosilane.

7. Procédé selon la revendication 6, dans lequel ledit verre, après liaison des acides nucléiques à celui-ci, est traité afin de désactiver la poly-L-lysine et/ou l'aminosilane.

8. Procédé selon la revendication 7, dans lequel ledit verre est traité avec une solution d'anhydride succinique en tant qu'agent bloquant et un catalyseur d'acylation dans un solvant non aqueux non polaire.

9. Procédé selon la revendication 8, dans lequel ledit catalyseur d'acylation est le N-méthylimidazole.

10. Procédé selon l'une quelconque des revendications 8 ou 9, dans lequel le solvant non aqueux non polaire est le 1,2-dichloroéthane.

11. Procédé selon l'une quelconque des revendications 8 à 10, dans lequel 0,2 g à 20 g d'anhydride succinique et 1 mL à 10 mL de N-méthylimidazole sont dissous dans environ 200 mL de 1,2-dichloroéthane.

12. Utilisation de bétaïnes en tant qu'additifs pour des solvants dans lesquels les acides nucléiques sont dissous afin de les lier à un support, dans laquelle les bétaines ont le groupement d'atome R₃N⁺-CH₂-COO⁻, dans lequel le groupe R est indépendamment choisi parmi un alkyle en C₁ à C₅.
